# EUROPÄISCHE PATENTSCHRIFT

(11) **EP 2 068 753 B1**
(45) Veröffentlichungstag und Bekanntmachung des Hinweises auf die Patenterteilung: **06.12.2017**
(21) Anmeldenummer: 07820631.5
(22) Anmeldetag: 26.09.2007
(51) Int. Cl.: A61C 8/00, A61K 6/093

(54) **ELASTISCHE PROVISORISCHE SUPRAKONSTRUKTION FÜR EIN DENTALES IMPLANTAT**
ELASTIC TEMPORARY SUPRACONSTRUCTION FOR A DENTAL IMPLANT
SUPERSTRUCTURE PROVISOIRE ÉLASTIQUE POUR IMPLANT DENTAIRE

(30) Priorität: 26.09.2006 DE 102006045466
(43) Veröffentlichungstag der Anmeldung: 17.06.2009
(73) Patentinhaber: VOCO GmbH, 27474 Cuxhaven (DE)
(72) Erfinder: SCHULZ-WALZ, Jan Erik, 22761 Hamburg (DE)
(74) Vertreter: Eisenführ Speiser
(86) Internationale Anmeldenummer: PCT/EP2007/060236
(87) Internationale Veröffentlichungsnummer: WO 2008/037753

(56) Entgegenhaltungen:
- WO-A-2006/089728
- DE-A1- 3 436 606
- DE-A1- 3 904 237
- DE-A1- 3 905 608
- DE-A1- 4 326 841
- US-A- 6 019 604
- US-A1- 2003 003 128
- US-A1- 2003 219 606
- "Silicones", 15 April 2003 (2003-04-15), ENCYCLOPEDIA OF POLYMER SCIENCE AND TECHNOLOGY, WILEY, US, PAGE(S) 765 - 841, XP007918236,

## Beschreibung

Die vorliegende Erfindung ist in den beigefügten Patentansprüchen definiert. Entsprechend betrifft die nachfolgende Beschreibung eine provisorische Suprakonstruktion für ein dentales Implantat mit einem Material mit einem Elastizitätsmodul von weniger als 300 MPa, die Verwendung eines solchen Materials zum Herstellen einer provisorischen Suprakonstruktion, ein Kit und ein Verfahren zum Herstellen einer provisorischen Suprakonstruktion sowie ein Abutment zur Befestigung einer provisorischen Suprakonstruktion. Vorteilhafte Ausführungsformen der Erfindung gehen aus den abhängigen Patentansprüchen hervor.

In der modernen Zahnmedizin stellt der Einsatz von Zahnimplantaten eine aufwendige, ansonsten jedoch zuverlässige und auch ästhetisch ansprechende Behandlungsmethode zur Versorgung von Zahnlücken dar.

Gegenüber den alternativen Möglichkeiten des Zahnersatzes wie festen nicht-implantatgestützten Brücken oder herausnehmbaren Prothesen weisen Implantate deutliche Vorteile auf; insbesondere belasten sie den Kieferknochen in fast der gleichen Weise wie der ursprüngliche Zahn. Ein zahnärztliches Implantat (dentales Implantat) ist eine künstliche Zahnwurzel meist in der technischen Ausgestaltung einer Schraube, die in den Kieferknochen eingepflanzt wird, wenn der Zahn mitsamt seiner Wurzel verlorengegangen ist. Durch das Verwachsen des Implantats mit dem Knochen übernimmt das Implantat fast die gleichen Funktionen wie die ursprüngliche Zahnwurzel und leitet ebenfalls Kräfte in den Knochen ein, wodurch der Knochen auf Zug beansprucht wird. Durch diese Belastung des Kieferknochens wird der Knochenstoffwechsel aktiv stimuliert und unterstützt, so dass der Kieferknochen erhalten bleibt, während er sich unter Brücken oder bei Prothesen mit der Zeit zurückbildet. Der zahnlose, nur vom Zahnfleisch bedeckte Kieferknochen unter Totalprothesen hingegen wird durch die fehlende auf Zug wirkende Krafteinleitung in den Knochen und zusätzlich oberflächlich wirkenden Druck falsch belastet und baut sich über Jahre teilweise langsam, teilweise rasant immer weiter ab. Durch den fortschreitenden Knochenabbau verändert sich das gesamte Knochenlager, was nach Jahren zu großen Problemen führt.

Zahnmedizinische Implantate werden daher in den Kieferknochen eingesetzt, um nach deren Einheilen eine im Vergleich zu einer Versorgung ohne Implantate bessere prothetische Versorgung für den Patienten zu ermöglichen und den ortständigen Knochen zu erhalten. So werden bei zahnlosen Patienten dadurch vielfach Totalprothesen vermieden, da der Patient entweder mit auf den Implantaten festsitzendem oder kombiniert festsitzend-herausnehmbarem Zahnersatz versorgt werden kann. Bei Lückengebissen kann das Beschleifen von gesunden Zahnen zur Herstellung von konventionellen (nicht-implantatgestützten) Brücken vermieden werden.

Der implantologische Eingriff ist recht umfangreich und umfasst im Wesentlichen die folgenden Schritte:
- Einsetzen des Implantats in den Kieferknochen
- Einheilung des Implantats in den Knochen
- Freilegung des Implantates
- Abdrucknahme mit speziellen Abdruckhilfen
- Herstellung des Zahnersatzes (der Suprakonstruktion)
- Einprobe des von der Zahntechnik angefertigten Zahnersatzes
- Einsetzen des endgültigen Zahnersatzes (endgültige Versorgung des Implantats)

Unter "Suprakonstruktion" wird im Rahmen des vorliegenden Textes ein Element verstanden, das direkt oder indirekt mit einem dentalen Implantat zu verbinden oder verbunden ist und dazu vorgesehen ist, vollständig oder fast vollständig in die Mundhöhle zu ragen. In der Regel ist die Suprakonstruktion ein Zahnersatz. Eine Suprakonstruktion kann beispielsweise eine Krone, eine Brücke (bzw. ein Teil einer Brücke) oder eine herausnehmbare Prothese (bzw. ein Teil einer herausnehmbaren Prothese) sein. Der Begriff "endgültig" wie etwa im Kontext "endgültige Suprakonstruktion" oder "endgültiger Zahnersatz" bezeichnet im Rahmen des vorliegenden Textes ein Element, das nicht von vornherein dazu bestimmt ist, nach einer gewissen Zeitspanne durch eine anderes Element ersetzt zu werden. Im Gegensatz dazu wird unter "Provisorium" im Rahmen des vorliegenden Textes ein Element verstanden, das von vornherein dazu bestimmt ist, zu einem späteren Zeitpunkt durch ein anderes Element ersetzt zu werden. Das Adjektiv "provisorisch" ist entsprechend zu verstehen. In der Regel ist ein Verbindungselement zwischen Implantat und Suprakonstruktion vorgesehen, ein sogenanntes Abutment (Aufbau). Ein gegebenenfalls vorhandenes Abutment wird im Rahmen des vorliegenden Textes nicht zur Suprakonstruktion gezählt. Unter "Verbinden" einer provisorischen Suprakonstruktion mit einem dentalen Implantat ist sowohl ein Positionieren der Suprakonstruktion direkt auf dem Implantat (ohne Verwendung eines Abutments) als auch ein Positionieren der Suprakonstruktion auf einem Abutment, das auf dem Implantat positioniert ist (indirektes Verbinden) zu verstehen.

Im oben angegebenen Behandlungsablauf beträgt die Einheilphase in der Regel zwischen 2 und 6 Monaten. Hierbei kommt es zur sogenannten Osseointegration des Implantates. Das bedeutet, dass Knochen auf die Implantatoberfläche aufwächst und das Implantat fest im Knochen verankert wird. Es kommt dabei aber noch nicht zu einer optimalen architektonischen Ausrichtung der Knochenstruktur, welche Kräfte aufnehmen oder zumindest optimal aufnehmen und optimal weiterleiten kann. Im oben angegebenen Behandlungsablauf wird bei der anschließenden Freilegung des Implantates und durch die darauf anschließend erfolgende Versorgung der Implantate mit der prothetischen Arbeit (der Suprakonstruktion) eine schlagartige einhundertprozentige Übertragung von Kräften, also eine ohne Übergangsphase einsetzende vollständige Übertragung der beim Kauen erzeugten Kräfte, auf das vorher durch Kräfte unbelastete Implantat und die unbelastete umgebende Knochenstruktur bewirkt. Der Nachteil des oben angegebenen Behandlungsablaufes ist also die sofortig einsetzende, einhundertprozentige Belastung der Implantate und umgebenden Knochenstruktur, welche eventuell nicht der Knochensituation um das Implantat herum entspricht und den Knochen in manchen Situationen überbelastet.

Um eine gute Osseointegration zu erreichen, benötigen der Knochen und die darin vorhandenen Zellen Zeit, auf der Oberfläche des Implantates anzuwachsen und umgebenden Knochen umzubauen. Bisher ging man davon aus, dass die Zeit und die Qualität des Knochens für die Einheilungsgeschwindigkeit und auch für den Erfolg der Osseointegration verantwortlich sind. Es wird diskutiert, ob auf das Implantat einwirkende physiologische Kräfte die Osseointegration fördern. Physiologische Kräfte würden die erstehende Knochenstruktur nur soweit belasten, wie es der bis dahin eingetretene Heilungsprozess erlaubt. Durch eine solche physiologische Krafteinleitung und eine daraus resultierende Verbesserung des Einheilvorganges lässt sich vermutlich die Einheilzeit verkürzen.

Wie lange ohne Anwendung einer solchen Technik der physiologischen Krafteinleitung die Einheilzeit sein soll, richtet sich nach anerkannten Klassifizierungen der Dichte und Struktur des Knochens, die Empfehlungen für bestimmte Einheilzeiten vorgeben. Im Allgemeinen sind dies für den Oberkiefer 6 Monate und für den Unterkiefer 3 Monate, da hier verschiedene Knochenstrukturen und Qualitäten zu finden sind.

Eine für die erfolgreiche Implantation weitere wichtige Voraussetzung ist, dass das Implantat nach dem Einbringen in den Knochen eine sogenannte Primärstabilität besitzt. Das heißt, dass es direkt nach dem Einbringen nicht locker sitzen darf, sondern absolut, d.h. im maximal möglichen Ausmaß, fest im Knochen verankert ist, was man auch mit Hilfe von Geräten wie den unter dem Namen Periotest® vertriebenen messen kann. Interessanterweise wird das Implantat nach initialer Primärstabilität oft nach etwa 14 Tagen etwas lockerer, um dann wieder fester zu werden. Dies wird durch Knochenab- und Umbauvorgänge erklärt. Wahrscheinlich ist das Implantat innerhalb dieser Zeit gegenüber äußeren Krafteinflüssen sehr sensibel.

In den letzten Jahren sind neue Überlegungen zu den nach dem Einsetzen eines Implantats erforderlichen Einheilzeiten gemacht worden. Vor allem dem Wunsch der Patienten folgend, nicht mehr so lange bis zur endgültigen Versorgung warten zu müssen, wurde angefangen, mit verkürzten Einheilzeiten oder ganz ohne Einheilzeiten und entsprechend der soeben genannten Alternative mit sofortiger (direkt nach der Implantation oder innerhalb von 24 bis 36 Stunden danach) provisorischer oder endgültiger Versorgung des Implantates und Belastung zu arbeiten.

Studien zur Sofortbelastung von Implantaten im Vergleich zu der herkömmlichen Methode sind widersprüchlich. Es gibt Studien, die zeigten, dass die erreichte Einheilung schlechter bei der Sofortimplantation war (M. Lorenzoni, C. Pertl, K. Zhang, W. Wegschneider, Clin. Oral Implants Res., 2003, 14 (3) 273-279); andere Studien fanden keinen Unterschied (P. Quinlan et al., Int. J. Oral Maxillofac Implants 2005, 20 (3), 360-370) oder kamen zu der Einschränkung, dass die Patienten genau auszuwählen und die Belastungskräfte auf die Implantate möglichst gering zu halten sind (G. Romanos, J. Oral Implantol. 2004, 30 (3), 189-197).

Die Misserfolge bei der Sofortbelastung scheinen durch zu frühe und zu starke Kraftübertragungen auf die Implantat-Knochenstruktur, die in diesen Fällen gerade im Aufbau und Umbau ist, herzurühren. Wahrscheinlich kann dieses System bestimmte physiologische Kräfte tolerieren, eventuell wirken diese sogar stimulierend; werden sie aber zu groß, reißt die Toleranz ab und es kommt zu keiner Osseointegration, sondern zu einer bindegewebigen Einscheidung des Implantates.

Es wurde gezeigt, dass eine physiologische Stimulierung der Osseointegration durch reduzierte Kaukräfte das Ergebnis verbessert (G Romanos et al., J. Periodontol. 2003, 74 (10), 1483-1490).

Diesen Erkenntnissen folgend wurde die sogenannte Methode der progressiven Kraftbelastung (progressive bone loading) beschrieben (R. Appleton et al., Clin. Oral Implants Res. 2005, 16 (2), 161-167). Dabei müssen die Kräfte langsam auf den Knochen übertragen werden. Diese steigende Kraftübertragung nennt man "progressives Belasten". Hierbei wird zunächst ein Provisorium (eine provisorische Suprakonstruktion) auf Kunststoffbasis eingesetzt, das zunächst sicherstellt, dass der provisorische Zahnersatz (das Provisorium) nicht mit seinem Antagonisten okkludiert. Der Patient ist in dieser Zeit angehalten, nur flüssige oder breiartige Nahrung zu sich zu nehmen Das Provisorium wird schließlich so abgeändert, dass es in mehreren Schritten immer dichter an den antagonistischen Zahn gebracht wird, bis es schließlich in Okklusion mit dem Gegenkiefer steht. Der Kieferknochen soll sich während dieses Zeitraums der steigenden Belastung anpassen, bis schließlich die endgültige Versorgung erfolgt.

Das dadurch erfolgende Knochentraining soll langsame, durch physiologische und reduzierte Kräfte induzierte Umbauvorgänge der Knochenarchitektur bewirken. Gerade diese Knochenarchitektur ist wichtig, um Kräfte im Knochen physiologisch zu verteilen, da dies ein Grundprinzip der Natur und des Knochens ist.

Der provisorische Zahnersatz wird im Stand der Technik aus herkömmlichen dentalen Prothesenkunststoffen angefertigt. Hierzu werden bevorzugt radikalisch polymerisierbare Acrylate eingesetzt. Diese Systeme sind in der Literatur erschöpfend beschrieben, so z.B. in der EP 270915, in der EP 630640, in der US 6063830, in der US 5548001, in der US 4617327, sowie in der EP 0677286. Neben den auf der Acrylatchemie basierenden Systemen wurden Spiroorthoester und polycyclische Ketallactone zur Herstellung von Dentalmaterialen vorgeschlaqen (US 4387215). Die DE 19506222 beschreibt kationisch polymerisierbare Materialien auf der Basis von Oxetan- und Oxacyclobutan-Derivaten. Weiterhin sind bicycloaliphatische 2-Methylen-1,3-dioxepane aus der DE 4439485 bekannt. Die US 5665839 offenbart radikalisch polymerisierbare Oxathiepane, die DE 19612004 beschreibt radikalisch polymerisierbare multifunktionelle Vinylcyclopropanderivate, während die DE 102004002178 Monomeren vorschlägt, die durch ringöffnende Metathese vernetzen können.

US 2003/0219606 A1 betrifft einen Siliconkautschuk mit verbesserten Hafteigenschaften (s. Abstract) und offenbart u.a. ein Zahnimplanat, an dem Siliconkautschuk angebracht ist (s. Abschnitt [0031] in Verbindung mit Figur 16). Das in Fig. 16 gezeigte Zahnimplanat (22) umfasst einen Silikonkautschuk (9H), welcher als "Schockabsorber" zwischen der Zahnprothese (24) und dem Implanatkörper (23) diene (vgl. Abschnitt [0050]). Als spezifisches Silicon zur Herstellung des Siliconkautschuks wird Realistic Variable Durometer (A-588) von Dow Corning Corp., U.S., und Silskin Clear von Silskin Corp., U.K., offenbart (vgl. Abschnitt [0039]). Platin wird zudem als Katalysator zur Polymerisation von Siliconen offenbart (vgl. [0038]). Das Vernetzen findet während der Polymerisation der Silicone statt (vgl. auch [0038]).

Allen diesen Systemen ist zu eigen, dass der resultierende dentale Formstoff grundsätzlich duroplastischen Charakter aufweist.

Die herkömmliche Methode mit den vergleichsweise langen Einheilzeiten hat den Nachteil, dass es nicht langsam und kontinuierlich zu diesen Umbauvorgängen kommt, da der Kieferknochen zunächst über einen längeren Zeitraum nicht belastet wird (keine Okklusion mit dem Gegenkiefer) und später eine plötzliche relative Vollbelastung durch das Provisorium bzw. die endgültige Versorgung (Suprakonstruktion) erfährt, welche auch zu Implantatverlusten führen kann.

Die Methode der Sofortbelastung ist nachteilig, denn sie birgt nach bisherigen Erkenntnissen das Risiko, dass es zu Überbelastungen der sich umbauenden Knochen-Implantatgrenze kommt, welche in dieser frühen Phase der Einheilung dann den Verlust des Implantates bedeuten können.

Nachteil der progressive bone loading Methode ist die Tatsache, dass Patienten nur Brei essen dürfen und dass es aufgrund der Tatsache, dass dann keine funktionellen Kaukräfte entstehen, zu keiner kontrollierbaren reduzierten Krafteinleitung im Vergleich zu einer Situation bei funktionaler Vollbelastung kommt. Falls der Patient jedoch Speisen zu sich nehmen sollte, die ein Kauen erfordern, sind die auf das Implantat einwirkenden Kaukräfte nicht zu kontrollieren.

Primäre Aufgabe der vorliegenden Erfindung war es, eine provisorische Suprakonstruktion für ein dentales Implantat anzugeben, mit deren Hilfe die mit der Verwendung herkömmlicher provisorischer Suprakonstruktionen jeweils verbundenen Nachteile minimiert werden können. Insbesondere sollte die provisorische Konstruktion eine Belastung des Implantats ermöglichen, insbesondere eine Belastung durch physiologische Kräfte wie oben definiert, die im Vergleich zur Situation bei Verwendung einer herkömmlichen provisorischen Suprakonstruktion besser kontrollierbar oder geringer ist.

Überraschenderweise hat sich gezeigt, dass diese Aufgabe gelöst werden kann durch eine provisorische Suprakonstruktion für ein dentales Implantat, gemäß Patentanspruch 1.

Der Begriff "Kern" bezieht sich hierbei auf ein Element, das dazu bestimmt ist, mit einer Kappe versehen zu werden oder das bereits mit einer Kappe versehen ist, wobei die Kombination aus Kern und Kappe eine provisorische Suprakonstruktion darstellt (zu näheren Einzelheiten siehe unten).

Ein Elastizitätsmodul von 300 MPa oder höher ist zum Erzielen einer physiologischen Krafteinleitung unvorteilhaft. Vorzugsweise sollte der Elastizitätsmodul im Bereich von 15 bis 200 MPa liegen, insbesondere im Bereich von 15 bis 150 MPa. Der Elastizitätsmodul wird hierbei bei 23°C, 50% relativer Luftfeuchtigkeit und 1 mm/min Vorschubgeschwindigkeit gemessen. Das erfindungsgemäß einzusetzende Material ist also elastisch und verglichen mit gesunder Zahnhartsubstanz (E-Moduln: Zahnschmelz 75 bis 90 GPa, Dentin 10 bis 20 GPa) relativ nachgiebig (weich, wenig steif). Die Elastizität sollte für die Dauer der Anwendung bestehen bleiben. Solche bevorzugten Materialien werden auch als dauerelastisch bezeichnet. Vorzugsweise weist eine erfindungsgemäße provisorische Suprakonstruktion ein besonders hohes Rückstellvermögen, also eine Fähigkeit, nach Verformung wieder in die ursprüngliche Form zurückzukehren, auf. Die Dimensionsstabilität gemäß ISO 4823:2000 sollte vorzugsweise 99% oder höher sein.Wie bereits erläutert, bezeichnet der Begriff "provisorische Suprakonstruktion" eine Suprakonstruktion, die von vornherein dazu bestimmt ist, zu einem späteren Zeitpunkt durch eine andere Suprakonstruktion ersetzt zu werden. In der Regel ist eine solche provisorische Suprakonstruktion dazu vorgesehen, einige Tage, Wochen oder Monate im Mund zu verbleiben, bis die Versorgung mit einer endgültigen Suprakonstruktion erfolgt. Die erfindungsgemäße provisorische Suprakonstruktion kann direkt nach der Implantation (Sofortbelastung) oder nach einer verkürzten oder herkömmlichen Einheilzeit (z.B. 3 bis 6 Monate) mit dem dentalen Implantat verbunden werden, vorzugsweise so, dass die provisorische Suprakonstruktion in Okklusion mit dem Gegenkiefer steht. Die provisorische Suprakonstruktion kann so gewährleisten, dass Kaukräfte aufgenommen und aufgrund der Biegsamkeit und Flexion der provisorischen Suprakonstruktion reduziert auf das Implantat weitergegeben werden. Vorzugsweise stellen während der Einheilphase verschiedene provisorische Suprakonstruktionen ansteigender Härte sicher, dass die Kontaktfläche zwischen Implantat und Kieferknochen zunehmenden Belastungen unterworfen wird. Die Patienten müssen sich nicht auf Brei beschränken, sondern können ihre Nahrung noch der funktionellen Ess- und Kaufunktion ihrer provisorischen Suprakonstruktion selbst bestimmen. Der Einsatz einer erfindungsgemäßen provisorischen Suprakonstruktion ist zudem aus ästhetischen Gründen vorteilhaft, da keine Zahnlücke auftritt.

Bevorzugt ist eine erfindungsgemäße provisorische Suprakonstruktion oder ein Kern für eine erfindungsgemäße provisorische Suprakonstruktion, wobei die provisorische Suprakonstruktion eine provisorische Krone ist. Unter "Krone" wird im Rahmen des vorliegenden Textes ein Element verstanden, das zum Ersatz eines einzelnen Zahnes bestimmt ist und dessen Retention auf einem anderen Element im Wesentlichen auf einem Formschluss oder Kraftschluss zwischen (zumindest einem Teil) seiner inneren Oberfläche und einer äußeren Oberfläche des anderen Elements beruht. Vorzugsweise ist eine Krone der natürlichen Zahngeometrie angenähert. Hierdurch ist eine kontrollierte Krafteinleitung besonders gut möglich. Eine erfindungsgemäße provisorische Suprakonstruktion kann jedoch auch ein provisorischer Brückenpfeiler sein.

Bei einer erfindungsgemäßen provisorischen Suprakonstruktion bzw. einem bevorzugten Kern weist das einzusetzende Material eine Shore A-Härte im Bereich von 20 bis 90 oder eine Shore D-Härte im Bereich von 30 bis 50 auf.

Vorzugsweise ist bei einer erfindungsgemäßen provisorischen Suprakonstruktion bzw. einem bevorzugten Kern das einzusetzende Material herstellbar durch Härtung eines härtbaren Ausgangsmaterials. Vorzugsweise ist die Härtung eine Selbsthärtung. Unter Selbsthärtung wird im Rahmen des vorliegenden Textes eine Härtung verstanden, die (a) keine im Vergleich zur Raumtemperatur wesentlich erhöhte Temperatur (z.B. 65°C und höher) (ein entsprechendes Ausgangsmaterial wird auch als kalthärtend bezeichnet) und (b) keine Bestrahlung mit elektromagnetischen Wellen (z.B. sichtbarem Licht oder Mikrowellen) erfordert.

Eine erfindungsgemäße provisorische Suprakonstruktion oder ein entsprechender Kern ist unter anderem dadurch charakterisiert, dass das härtbare Ausgangsmaterial aus folgenden Bestandteilen besteht oder diese umfasst:
(a) ein oder mehrere Silicone umfassend mehratomige vernetzbare Gruppen, vorzugsweise ungesättigte vernetzbare Gruppen, und/oder einen oder mehrere gegebenenfalls substituierte, vernetzbare Polyether,
(b) einen oder mehrere Katalysatoren oder Starter für die Vernetzung,
(c) optional einen oder mehrere Vernetzer, für die gilt: der oder die Vernetzer sind keine Silicone umfassend mehratomige vernetzbare Gruppen und keine gegebenenfalls substituierten Polyether,
   und
(d) optional einen oder mehrere anorganische oder organische Füllstoffe.

Mehratomige vernetzbare Gruppen sind Gruppen, die (i) eine Reaktion eingehen können, mit deren Hilfe Moleküle des einen oder der mehreren Silicone des Bestandteils (a) miteinander und/oder mit anderen Bestandteilen, insbesondere Bestandteil (c), kovalent verknüpft werden können und die (ii) nicht lediglich aus einem Atom (z.B. H) bestehen. Es ist erfindungsgemäß möglich und in manchen Fällen vorteilhaft, Silicone des Bestandteils (a) einzusetzen, bei denen mehratomige vernetzbare Gruppen und Si-H-Bindungen in einem Molekül vorliegen. Insbesondere in einem solchen Fall kann Bestandteil (c) entfallen.

Katalysatoren des Bestandteils (b) werden insbesondere eingesetzt, falls Bestandteil (a) Silicone umfasst; und Starter des Bestandteils (b) insbesondere, falls Bestandteil (a) Polyether umfasst. Katalysatoren bzw. Starter bewirken eine Vernetzung der vernetzbaren Gruppen des Bestandteils (a), gegebenenfalls unter Einbeziehung vernetzbarer Gruppen eines Vernetzers gemäß Bestandteil (c). Ein Vernetzer gemäß Bestandteil (c) umfasst vernetzbare Gruppen, die zur Umsetzung mit vernetzbaren Gruppen des Bestandteils (a) vorgesehen sind.

Als Füllstoffe des Bestandteils (d) sind grundsätzlich alle inerten Materialien geeignet. Vorzugsweise haben die Füllstoffe einen Einfluss auf den E-Modul und/oder die Härte und/oder beeinflussen die Konsistenz des Ausgangsmaterials. Beispiele für anorganische Füllstoffe sind Gläser, Siliciumdioxid (z.B. als Quarze, Cristobalite, pyrogenes SiO₂ wie etwa das unter dem Namen AEROSIL® (Degussa) angebotene und/oder nach einem Sol-Gel-Verfahren hergestelltes SiO₂), Beispiele für organische Füllstoffe sind Acrylate. Die Füllstoffe werden während der Abreaktion der reaktiven Bestandteile (a) sowie gegebenenfalls (b) und/oder (c) in die Matrix eingebaut und verleihen dem gehärteten Material Festigkeit.

Vorzugsweise ist das Material, aus dem die erfindungsgemäße provisorische Suprakonstruktion besteht, gefärbt, insbesondere um seine Farbe an eine Farbe natürlicher Zähne anzupassen. Beispiele für farbgebende Bestandteile sind z.B. ein oder mehrere anorganische oder organische Füllstoffe des Bestandteils (d) (z.B. zahnfarbene Acrylat-Partikel und/oder gemahlener Quarz. Es können jedoch auch zusätzliche farbgebende Bestandteile als optionale Komponente (e) vorgesehen sein, beispielsweise kleine organische Farbstoffe wie etwa Diazo-Farbstoffe.

Der Einsatz von Siliconen und Polyethern im dentalen Bereich ist bekannt.

Am häufigsten werden kondensationsvernetzende hydroxylgruppenhaltige oder additionsvernetzende vinylgruppenhaltige Silicone sowie Polyethermaterialien als dentale Abformmassen eingesetzt. Bekannt ist der Einsatz von Siliconen bislang zum Füllen und zum Verschluss von Wurzelkanälen (DE 3915592, US 3082527 und DE 19709531). Darüber hinaus beschreibt EP 1307174 die Verwendung von Siliconen als haftendes und provisorisches Zahnfüllungsmaterial, als haftenden und provisorischen Zement sowie als Adhäsiv.

Bisher unbekannt ist der Einsatz von Siliconen und Polyethern zur Herstellung von provisorischem Zahnersatz.

Besonders bevorzugt ist eine erfindungsgemäße provisorische Suprakonstruktion oder ein Kern, wobei das härtbare Ausgangsmaterial aus folgenden Bestandteilen besteht oder diese umfasst:
(i)
   (a) ein oder mehrere Silicone umfassend (als bevorzugte vernetzbare Gruppen) Alkenylgruppen, die gegebenenfalls substituiert sind,
   (b) einen oder mehrere Katalysatoren für die Vernetzung,
   (c) ein oder mehrere Organohydrogenpolysiloxane ohne mehratomige vernetzbare Gruppen, vorzugsweise ein oder mehrere Alkylhydrogenpolysiloxane (als bevorzugte Vernetzer für Bestandteil (a)), und
   (d) einen oder mehrere partikuläre anorganische oder organische Füllstoffe, vorzugsweise ausgewählt aus der Gruppe bestehend aus Siliciumdioxid, Metalloxiden und Acrylaten,
      oder
(ii)
   (a) Alkylpolysiloxan mit endständigen Hydroxylgruppen,
   (b) einen oder mehrere Katalysatoren,
   (c) ein oder mehrere Silane mit zwei oder mehr Alkoxysubstituenten, und
   (d) einen oder mehrere partikuläre anorganische oder organische Füllstoffe, vorzugsweise ausgewählt aus der Gruppe bestehend aus Siliciumdioxid, Metalloxiden und Acrylaten, oder
(iii)
   (a) ein oder mehrere Polyalkylenglycole, bei denen die endständigen OH-Gruppen durch Aziridin umfassende Gruppen ersetzt sind, wobei vorzugsweise das eine oder mindestens eines der mehreren entsprechenden Polyalkylenglycole eine einzige oder nur zwei unterschiedliche repetitive Einheiten mit zwei bis vier C-Atomen aufweist,
   (b) einen oder mehrere Starter, und
   (d) einen oder mehrere partikuläre anorganische oder organische Füllstoffe, vorzugsweise ausgewählt aus der Gruppe bestehend aus Siliciumdioxid, Metalloxiden und Acrylaten.

Alternative (i) bezieht sich auf additionsvernetzende Silicone. Als kalthärtende und selbsthärtende Silicone sind sie besonders gut geeignet zur Herstellung der erfindungsgemäßen provisorischen Suprakonstruktionen, da sie eine sehr geringe Schrumpfung beim Härten aufweisen. Die Härtung ist hierbei eine Härtung, die durch Hydrosilylierung erfolgt, eine Additionsreaktion von Organohydrogenpolysiloxanen (Polysiloxanen, die zum einen organische Gruppen und zum anderen Si-H-Bindungen aufweisen) des Bestandteils (c) an Silicone umfassend (gegebenenfalls substituierte) ungesättigte Kohlenwasserstoffgruppen des Bestandteils (a), in der Regel (gegebenenfalls substituierte) Alkenylgruppen, bevorzugt Vinyl- oder Allylgruppen.

Es ist aus Gründen der Elastizität bevorzugt, dass eines oder mehrere der Silicone des Bestandteils (a) umfassend gegebenenfalls substituierte Alkenylgruppen linear (d.h. im Siloxangrundgerüst unverzweigt) ist. Bei einem solchen linearen Silicon sind pro Molekül zwei der (gegebenenfalls substituierten) Alkenylgruppen, insbesondere Vinyl- oder Allylgruppen, an den Kettenenden (terminal) angeordnet. Optional befinden sich zusätzliche solcher Gruppen zur Erhöhung des Vernetzungsgrades inmitten der Kette (d.h. nicht terminal), bevorzugt jedoch nicht eine zu hohe Anzahl, etwa pro Molekül ein oder zwei oder sogar überhaupt keine zusätzliche solcher Gruppen inmitten der Kette, da durch sie das gehärtete Material regelmäßig rigider und somit spröder wird. Vorteilhaft ist oftmals auch der Einsatz von verzweigten Siliconen umfassend gegebenenfalls substituierte Alkenylgruppen in Bestandteil (a). Besonders vorteilhaft ist es, zusätzlich zu linearen Siliconen VQM-Silicone einzsetzen (vinylterminierte quartär modifizierte Silicone). Es ist bevorzugt, wenn diese aus Molekülen mit je einem Siliciumatom bestehen, das mit vier Siloxanketten substituiert ist, an deren Ende sich jeweils eine Vinylgruppe befindet. Solche Silicone führen zu einem Gewinn an Härte des gehärteten Materials, ohne dass ein großer Flexibilitätsverlust oder eine große Sprödigkeit in Kauf genommen werden muss und stellen somit gegebenenfalls eine Alternative zu Füllstoffen des Bestandteils (d) dar, die in manchen Fällen die Sprödigkeit erhöhen.

Vorzugsweise sind die Organohydrogenpolysiloxane des Bestandteils (c) Alkylhydrogenpolysiloxane (Polysiloxane, die zum einen Alkylgruppen und zum anderen Si-H-Bindungen aufweisen), wobei die Alkylgruppen bevorzugt jeweils 1 bis 4 C-Atome aufweisen und besonders bevorzugt Methylgruppen sind. Organohydropolysiloxane mit mindestens drei Si-H-Bindungen pro Molekül werden als Vernetzer eingesetzt. Zusätzlich können auch Organohydrogenpolysiloxane mit zwei Si-H-Bindungen pro Molekül als sogenannte Kettenverlängerer zur Beeinflussung des Aushärteverhaltens und der mechanischen Festigkeit eingesetzt werden.

Vorzugsweise weisen die Organohydrogenpolysiloxane des Bestandteils (c) pro Molekül 2 bis 3 Si-H-Bindungen auf. Bevorzugt sind hierbei terminale Si-H-Bindungen, optional können sie aber auch zusätzlich oder ausschließlich inmitten der Kette vorliegen. Organohydrogenpolysiloxane, die Si-H-Bindungen inmitten der Kette aufweisen, sind vorzugsweise vergleichsweise kurz und werden dann als "Modifier" bezeichnet.

Alternative (ii) bezieht sich auf kondensationsvernetzende (kalthärtende und selbsthärtende) Silicone. Hierbei werden Alkylpolysiloxane mit endständigen Hydroxylgruppen des Bestandteils (a) durch Silane mit zwei oder mehr Alkoxysubstituenten des Bestandteils (c) unter Abspaltung eines Alkohols miteinander vernetzt. Erfindungsgemäß möglich sind zwar auch Alkylpolysiloxane des Bestandteils (a) mit Hydroxylgruppen inmitten der Kette; diese sind jedoch weniger bevorzugt, da sie in der Regel die Sprödigkeit des gehärteten Materials ungünstig beeinflussen. Die Alkylgruppen der Alkylpolysiloxane des Bestandteils (a) weisen vorzugsweise jeweils 1 bis 4 C-Atome auf; Methylgruppen sind besonders bevorzugt. Die Alkoxysubstituenten der Silane des Bestandteils (c) besitzen vorzugsweise jeweils 1 bis 4 C-Atome und sind besonders bevorzugt Ethoxy. In letzterem Fall wird bei der Vernetzung Ethanol abgespalten.

Alternative (iii) bezieht sich auf (derivatisierte) Polyether. Zur Bildung des Aziridin-Derivats eines Polyalkylenglycols gemäß Bestandteil (a) werden die endständigen OH-Gruppen eines Polyethers (Polyalkylenglycols) mit ungesättigten Säuren verestert und die resultierenden Ester anschließend mit Aziridin (Ethylenimin) umgesetzt. An den Kettenenden entstehen so reaktionsfreudige Aziridin-Gruppen. Aziridin ist ein sehr reaktiver dreigliedriger heterozyklischer Ring und lässt sich leicht öffnen. Durch die Gegenwart eines Starters (Bestandteil (b)) kommt es zur Polyaddition und die Bestandteile reagieren zum vernetzten Endprodukt ab.

Ganz besonders bevorzugt ist eine provisorische Suprakonstruktion oder ein Kern wie vorstehend definiert (vorzugsweise in einer der vorstehend als bevorzugt bezeichneten Ausgestaltungen), wobei das härtbare Ausgangsmaterial aus folgenden Bestandteilen besteht oder diese umfasst:
(i)
   (a) zwei oder mehr Silicone, die jeweils sowohl (1) vernetzbare Gruppen ausgewählt aus der Gruppe bestehend aus Vinyl und Allyl als auch (2) Alkylgruppen umfassen, wobei die Silicone vorzugsweise unterschiedlich beschaffene Vinylmethylpolysiloxane sind,
   (b) einen oder mehrere Katalysatoren vorzugsweise umfassend Platin oder Palladium,
   (c) Methylhydrogenpolysiloxan, und
   (d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe,
   oder
(ii)
   (a) Polydimethylsiloxan mit endständigen Hydroxylgruppen,
   (b) Zinnoctoat und/oder Dibutylzinnlaurat,
   (c) ein oder mehrere Silane mit zwei oder mehr Ethoxy-Substituenten, und
   (d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe,
   oder
(iii)
   (a) ein oder mehrere Polyalkylenglycole, bei denen die endständigen OH-Gruppen durch Aziridin umfassende Gruppen ersetzt sind und die zwei unterschiedliche repetitive Einheiten mit zwei bzw. vier C-Atomen aufweisen,
   (b) einen oder mehrere kationische Starter, und
   (d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe.

Im Fall der additionsvernetzenden Silicone (Alternative (i)) sind Silicone des Bestandteils (a) bevorzugt, die sowohl vernetzbare Gruppen ausgewählt aus der Gruppe bestehend aus Vinyl und Allyl als auch Alkylgruppen umfassen, vorzugsweise Alkylgruppen mit jeweils 1 bis 4 C-Atomen, insbesondere Methyl. Ganz besonders bevorzugt sind Vinylmethylpolysiloxane. Diese weisen terminal Dimethylvinylsiloxaneinheiten auf und besitzen vorzugsweise ein mittleres Molekulargewicht im Bereich von 2500 bis 350000 g/mol. Besonders bevorzugt ist der Einsatz mehrerer unterschiedlich beschaffener Silicone, insbesondere Vinylmethylpolysiloxane, die z.B. unterschiedliche Viskositäten aufweisen, beispielsweise im Bereich von etwa 200, 1000 und 10000 mPa*s, oder die sich hinsichtlich ihrer mittleren Molekulargewichte unterscheiden. Zwei Silicone, deren Molekulargewichtsverteilung jeweils nur ein Maximum aufweist, unterscheiden sich z.B. hinsichtlich ihrer mittleren Molekulargewichte, wenn die Molekulargewichtsverteilung ihrer Mischung zwei Maxima aufweist.

Die oberflächenmodifizierten oder nicht-oberflächenmodifizierten SiO₂-Partikel eines mittleren Teilchendurchmessers von unter 200 nm des Bestandteils (d) sind vorzugsweise pyrogenes SiO₂ wie es etwa unter dem Namen AEROSIL® (Degussa) angeboten wird. Diese Teilchen beeinflussen sowohl die Härte des gehärteten Materials als auch die Konsistenz des härtbaren Ausgangsmaterials und sind vorzugsweise oberflächlich modifizert. Die in Bestandteil (d) optional weiter enthaltenen partikulären anorganischen oder organischen Füllstoffe sind vorzugsweise siliciumhaltig und umfassen z.B. gemahlenen Quarz, beispielsweise eines mittleren Teilchendurchmessers von ca. 10 µm. Im Fall der Alternativen (ii) und (iii) gilt für Bestandteil (d) ebenfalls das soeben Gesagte.

Bevorzugte Katalysatoren (Bestandteil (b)) für die Vernetzung der kondensationsvernetzenden Silicone (Alternative (ii)) sind Zinnoctoat und/oder Verbindungen, bei denen ein Zinnatom an zwei Alkylgruppen gebunden ist, vorzugsweise Butyl, wie etwa Dibutylzinnlaurat.

Bei Alternative (iii) sind die als Polyalkylenglycole des Bestandteils (a) längerkettige lineare Copolymere aus Ethylenoxid- und Butylenoxideinheiten bevorzugt, bei denen die endständigen OH-Gruppen durch Aziridin umfassende Gruppen ersetzt sind.

Wie bereits oben beschrieben betrifft die vorliegende Erfindung auch eine provisorische Suprakonstruktion bestehend aus einem Kern wie vorstehend definiert (vorzugsweise in einer der vorstehend als bevorzugt bezeichneten Ausgestaltungen) und einer Kappe für den Kern, wobei das Material der Kappe einen höheren Elastizitätsmodul aufweist als das Material des Kerns. Die Kappe dient vorzugsweise dazu, eine Oberfläche mit gegenüber der Oberfläche des Kerns verbesserten Kaueigenschaften (insbesondere einer erhöhten Abrasionsfestigkeit) bereitzustellen. Material, Dicke und Form der Kappe sind so ausgewählt und an den Kern angepasst, dass die Fähigkeit des Kerns, Kaukräfte reduziert auf das Implantat zu übertragen, erhalten bleibt. Die Kappe ist vorzugsweise so beschaffen, dass sie nur einen Teil der äußeren Oberfläche des Kerns bedeckt, wobei dieser Teil die Kaufläche umfasst. Die Kappe besteht vorzugsweise aus Keramik oder aus einem üblichen provisorischen Kronenmaterial (z.B. Kunststoffen oder Compositen auf Kunststoffbasis, etwa auf Basis von Methacrylat-haltigen Verbindungen wie Polymethylmethacrylat (PMMA), Bisphenol-A-Glycidylmethacrylat (Bis-GMA), Urethandimethacrylat (UDMA) oder Triethylenglycoldimethacrylat (TEGDMA); siehe auch die obigen Ausführungen zu herkömmlichen dentalen Prothesenkunststoffen bzw. duroplastischen dentalen Formstoffen). Die Kappe wird auf einen zugeordneten Kern beispielsweise aufgeklebt oder unter Ausbildung eines Klemmsitzes (gegebenenfalls mit Formschluss) auf diesem angeordnet, um die provisorische Suprakonstruktion herzustellen.

Gemäß einem weiteren Aspekt betrifft die vorliegende Erfindung ein Kit zum Herstellen einer oder mehrerer erfindungsgemäßer provisorischer Suprakonstruktionen wie vorstehend definiert oder eines oder mehrerer Kerne wie vorstehend definiert (jeweils vorzugsweise in einer der vorstehend als bevorzugt bezeichneten Ausgestaltungen), umfassend oder bestehend aus
- Bestandteil (a), Bestandteil (b), Bestandteil (d) und optional Bestandteil (c) (wie oben als zusammengehörig definiert), wobei ein oder mehrere farbgebende Bestandteile zur Anpassung der Farbe der einen oder der mehreren provisorischen Suprakonstruktionen an jeweils eine Farbe natürlicher Zähne vorgesehen sind, oder
- ein oder mehrere vorgeformte Rohlinge bestehend aus einem gehärteten (Ausgangs-)Material wie vorstehend definiert, wobei die Farbe des einen bzw. der mehreren vorgeformten Rohlinge jeweils einer Farbe natürlicher Zähne angepasst ist.

Die farbgebenden Bestandteile können unterschiedlicher chemischer Natur sein. Bei einem bevorzugten erfindungsgemäßen Kit besteht Bestandteil (d) aus einem oder mehreren farbgebenden Füllstoffen als farbgebenden Bestandteilen oder umfasst diese. Beispiele dafür sind etwa zahnfarbene Acrylat-Partikel und/oder gemahlener Quarz. Es können jedoch auch zusätzliche farbgebende Bestandteile vorgesehen sein, beispielsweise kleine organische Farbstoffe wie etwa Diazo-Farbstoffe. Diese können auch in Mischung mit anderen Bestandteilen eingesetzt werden, etwa als LSR-Farbpaste (GE Bayer Silicones) in Mischung mit Divinylpolysiloxanen des Bestandteils (a).

Die Farbe der erfindungsgemäßen provisorischen Suprakonstruktion bzw. des vorgeformten Rohlings kann an eine gebräuchliche Farbskala für dentale Zwecke angelehnt sein. Ein Beispiel ist die von der VITA Zahnfabrik hergestellte Farbskala. Bevorzugte Farben sind A1, A2, A3, A3.5, B1, B3 und BL.

In einem erfindungsgemäßen Kit, das aus Bestandteil (a), Bestandteil (b), Bestandteil (d) und optional Bestandteil (c) besteht oder diese umfasst, sind diese wie oben als zusammengehörig definiert ausgewählt. Dies bedeutet beispielsweise, dass bei einer Auswahl der Bestandteile gemäß der vorstehend geschilderten Alternative (i) nicht nur Bestandteil (a), sondern auch die Bestandteile (b) und (d) und, sofern vorhanden, Bestandteil (c) gemäß Alternative (i) und nicht gemäß einer anderen Alternative ausgewählt sind.

Unter einem vorgeformten Rohling ist ein Vorläufer einer provisorischen Suprakonstruktion oder eines Kerns zu verstehen, der im Allgemeinen nicht in unveränderter Form mit einem Implantat verbunden werden kann, sondern dazu bestimmt ist, hinsichtlich seiner Form an eine bestimmte Patientensituation angepasst zu werden, beispielsweise durch Entfernen (z.B. Abschneiden, Abschleifen oder ein sonstiges abtragendes formgebendes Verfahren) von Überschüssen.

Bevorzugt ist ein erfindungsgemäßes Kit zum Herstellen mehrerer (z.B. 2, 3 oder mehr) provisorischer Suprakonstruktionen oder Kerne, deren Materialien sich hinsichtlich ihrer Elastizitätsmoduln und/oder Werte der Shore A- bzw. Shore D-Härte unterscheiden. Diese sind regelmäßig dazu bestimmt, in der Reihenfolge ansteigender Elastizitätsmoduln bzw. Werte der Shore-Härte zeitlich nacheinander mit dem dentalen Implantat verbunden zu werden. Besonders bevorzugt ist ein erfindungsgemäßes Kit zum Herstellen von 3 oder mehr provisorischen Suprakonstruktionen oder Kernen, wobei jeweils eine oder mehrere provisorische Suprakonstruktionen bzw. Kerne aus Materialien mit Elastizitätsmoduln in den Bereichen (i) 15 bis 40 MPa, (ii) 55 bis 70 MPa und (iii) 100 bis 120 MPa und/oder aus Materialien mit einer Härte in den Bereichen (i) Shore A-Härte 25 bis 45, (ii) Shore A-Härte 75 bis 85 und (iii) Shore D-Härte 35 bis 45 bestehen.

Ein bevorzugtes erfindungsgemäßes Kit umfasst oder besteht aus Bestandteil (a), Bestandteil (b), Bestandteil (d) und optional Bestandteil (c), wobei das oder die härtbaren Ausgangsmaterialien für die eine oder die mehreren provisorischen Suprakonstruktionen oder Kerne jeweils aus einer ersten und einer zweiten Komponente anzumischen sind, wobei die erste und die zweite Komponente zusammen Bestandteil (a), Bestandteil (b), Bestandteil (d) und optional Bestandteil (c) umfassen oder daraus bestehen und so zusammengesetzt und aneinander angepasst sind, dass durch das Mischen der Komponenten das Ausgangsmaterial resultiert und dessen Härtung initiiert wird. Dies kann - abhängig von der Natur der gewählten Bestandteile - z.B. dadurch geschehen, dass die erste Komponente die Bestandteile (a) und (c), nicht aber Bestandteil (b) umfasst und die zweite Komponente die Bestandteile (a) und (b), nicht aber Bestandteil (c). Es ist dem Fachmann ohne weiteres möglich, beliebige andere Optionen zu wählen. Als weiteres Beispiel könnte die erste Komponente Bestandteil (a) und (b) umfassen, nicht aber Bestandteil (c) und die zweite Komponente die Bestandteile (c) und (d), nicht aber Bestandteil (b).

Die Dosierung der ersten und der zweiten Komponente kann auf unterschiedliche Weise erfolgen. Die Komponenten können z.B. in Tuben oder Flaschen zur Verfügung gestellt werden. Die Anmischung erfordert in einem solchen Fall eine visuell volumetrische Dosierung. Vorzugsweise sind bei einem erfindungsgemäßen Kit, bei dem das oder die härtbaren Ausgangsmaterialien für die eine oder die mehreren provisorischen Suprakonstruktionen jeweils aus einer ersten und einer zweiten Komponente anzumischen sind, die erste und die zweite Komponente im Volumenverhältnis von 10:1 bis 1:10, bevorzugt 4:1 bis 1:4, besonders bevorzugt 2:1 bis 1:2 und insbesondere 1:1 anzumischen.

Liegen die erste und die zweite Komponente beide in Form einer Paste vor (Paste-Paste-Systeme), so können die beiden Komponenten bei vorzugsweise gleicher Strangdicke über die Stranglängen (z.B. gleiche Stranglängen) entsprechend einer vorgegebenen Skalierung auf einem Anmischblock ausgedrückt werden. Eine der Komponenten kann jedoch auch in flüssiger Form vorliegen. Für diesen Fall kann sie über Messkappen oder mittels Tropfen einer Basispaste zugegeben werden.

Vorzugsweise wird die Dosierung mit Hilfe einer statischen Mischvorrichtung durchgeführt, z.B. automatisch mit Hilfe eines Kartuschensystems mit statischem Mischrohr. Ein bevorzugtes erfindungsgemäßes Kit umfasst ferner eine statische Mischvorrichtung. Diese umfasst vorzugsweise die folgenden Elemente: eine mit einer Doppelkammerkartusche oder mit zwei Schlauchbeuteln verbundene statische Mischdüse, eine von der statischen Mischdüse versorgte Öffnung sowie Mittel zum gleichzeitigen Auspressen des Inhalts der beiden Kammern bzw. Schlauchbeutel, wobei die eine Kammer bzw. der eine Schlauchbeutel die erste Komponente und die andere Kammer bzw. der andere Schlauchbeutel die zweite Komponente enthält.

Vorzugsweise sind in einem erfindungsgemäßen Kit wie vorstehend definiert (vorzugsweise in einer der vorstehend als bevorzugt bezeichneten Ausgestaltungen) die erste und die zweite Komponente so aneinander angepasst, dass nach dem Mischen die Vernetzung innerhalb eines Zeitraumes von 30 s bis 10 min abgeschlossen ist.

Alternativ umfasst ein erfindungsgemäßes Kit wie oben ausgeführt ein oder mehrere vorgeformte Rohlinge (mit oben angegebenen Eigenschaften) oder besteht daraus. Vorzugsweise ist deren Form bereits jeweils einer Form natürlicher Zähne angenähert, z.B. der Form von Schneidezähnen, Eckzähnen, Prämolaren und/oder Molaren.

Vorzugsweise umfasst ein erfindungsgemäßes Kit mehrere vorgeformte Rohlinge mit identischer oder unterschiedlicher Farbe oder besteht daraus.

Vorzugsweise umfasst ein erfindungsgemäßes Kit mehrere vorgeformte Rohlingen mit unterschiedlichen Elastizitätsmoduln und/oder Werten der Shore A- bzw. Shore D-Härte oder besteht daraus. Dies gilt auch für den Spezialfall eines Kits, das jeweils nur einen vorgeformten Rohling, bei dem eine Shore A-Härte gemessen werden kann, und einen vorgeformten Rohling, bei dem eine Shore D-Härte gemessen werden kann, umfasst.

Ein bevorzugtes erfindungsgemäßes Kit umfasst oder besteht aus 3 oder mehr vorgeformten Rohlingen, wobei jeweils ein oder mehrere vorgeformte Rohlinge Elastizitätsmoduln in den Bereichen (i) 15 bis 40 MPa, (ii) 55 bis 70 MPa und (iii) 100 bis 120 MPa. und/oder eine Härte in den Bereichen (i) Shore A-Härte 25 bis 45, (ii) Shore A-Härte 75 bis 85 und (iii) Shore D-Härte 35 bis 45 aufweisen. Diese Bereiche haben sich als besonders geeignet zur kontrollierten Krafteinleitung auf ein dentales Implantat herausgestellt. Werden entsprechende provisorische Suprakonstruktionen in der Reihenfolge ansteigender Elastizitätsmoduln zeitlich nacheinander mit dem dentalen Implantat verbunden, so kann eine besonders ausgewogene allmählich steigende Krafteinleitung (Belastung) erfolgen.

Die vorliegende Erfindung betrifft auch ein Kit umfassend oder bestehend aus mehreren (a) provisorischen Suprakonstruktionen wie vorstehend definiert, (b) vorgeformten Rohlingen für provisorische Suprakonstruktionen wie vorstehend definiert oder (c) Kombinationen aus jeweils einem Kern für eine provisorische Suprakonstruktion wie vorstehend definiert und einem zugeordneten Kappenrohling. Der Kappenrohling einer solchen Kombination ist dazu bestimmt, hinsichtlich seiner Form an eine bestimmte Patientensituation angepasst zu werden, beispielsweise durch Entfernen (z.B. Abschneiden, Abschleifen oder ein sonstiges abtragendes formgebendes Verfahren) von Überschüssen. Vorzugsweise sind in einem solchen Kit ein einzelner, mehrere oder sämtliche der (a) provisorischen Suprakonstruktionen, (b) vorgeformten Rohlinge bzw. (c) Kombinationen mit einem Abutment für ein dentales Implantat verbunden. Hinsichtlich der bevorzugten Ausgestaltungen der (a) provisorischen Suprakonstruktionen bzw. (b) Rohlinge gelten die vorstehenden Ausführungen entsprechend.

Ein weiterer Aspekt der vorliegenden Erfindung betrifft ein Abutment zur Befestigung einer erfindungsgemäßen provisorischen Suprakonstruktion wie vorstehend definiert (vorzugsweise in einer der vorstehend als bevorzugt bezeichneten Ausgestaltungen) umfassend oder bestehend aus den folgenden Elementen:
(A) ein Element zur Retention der erfindungsgemäßen provisorischen Suprakonstruktion,
(B) eine Plattform zur Abstützung der erfindungsgemäßen provisorischen Suprakonstruktion in Kraftrichtung entlang der Längsachse eines Implantats, an dem das Abutment befestigt oder zu befestigen ist zum Kieferknochen hin, in den das Implantat eingesetzt oder einzusetzen ist,
(C) ein Element, das als Gingivaformer ausgebildet ist,
wobei Element (A) und die provisorische Suprakonstruktion so aneinander angepasst sind, dass die provisorische Suprakonstruktion an Element (A) formschlüssig befestigt ist in Kraftrichtung entlang der Längsachse des Implantats vom Kieferknochen weg, in den es eingesetzt oder einzusetzen ist. Vorzugsweise wird dies dadurch erreicht, dass Element (A) eine oder mehrere Unterschneidungen aufweist, in die die provisorische Suprakonstruktion hineingreifen kann. Vorzugsweise wird bei der Herstellung eines Rohlings ein Unterschneidungen aufweisendes Element (A) oder eine vergleichbar geformte Struktur mit einem härtbaren Ausgangsmaterial umschlossen (z.B. umspritzt), das anschließend gehärtet wird.

Die erfindungsgemäße provisorische Suprakonstruktion kann jedoch auch unter Verwendung eines üblichen provisorischen Dentalzements oder allein durch eigene Friktion (Reibschluss) mit einem dentalen Implantat verbunden werden, optional unter Einsatz eines Abutments (in erfindungsgemäßer oder nichterfindungsgemäßer Ausgestaltung). Das Abutment besteht vorzugsweise aus Kunststoff oder Metall. Element (A) kann eine beliebige Form aufweisen; optional weist Element (A) auch einen oder mehrere Fortsätze (beispielsweise in der Art von Fingern) auf, die zur Aufnahme von Kräften in der Lage sind.

Bevorzugt ist ein Abutment, wobei Element (A) und die provisorische Suprakonstruktion so aneinander angepasst sind, dass die provisorische Suprakonstruktion an Element (A) formschlüssig befestigt ist gegen eine Drehkraft um die Längsachse des Implantats. Dies kann beispielsweise dadurch erreicht werden, dass Element (A) einen Umriss aufweist, der aus der Achse betrachtet, die in Funktion mit der Längsachse des Implantats übereinstimmt, nicht kreisförmig ist, sondern etwa oval, eiförmig, dreieckig (z.B. rechtwinklig dreieckig), rechteckig (z.B. quadratisch), anderweitig polygon oder anderweitig geformt, insbesondere mit möglichst geringer oder ohne Rotationssymmetrie, um ein Einsetzen und eine Retention in der richtigen Orientierung zu erleichtern.

Ein weiterer Aspekt der vorliegenden Erfindung betrifft eine Kombination aus einer erfindungsgemäßen provisorischen Suprakonstruktion mit oder ohne Kappe) wie vorstehend definiert (vorzugsweise in einer der vorstehend als bevorzugt bezeichneten Ausgestaltungen) und einem Abutment und/oder einem dentalen Implantat.

Weiterhin betrifft die vorliegende Erfindung ein Verfahren zum Herstellen einer erfindungsgemäßen provisorischen Suprakonstruktion wie vorstehend definiert oder eines Kerns wie vorstehend definiert (jeweils vorzugsweise in einer der vorstehend als bevorzugt bezeichneten Ausgestaltungen) umfassend die Schritte:
- Bereitstellen einer geeigneten Form,
- Bereitstellen eines härtbaren Ausgangsmaterials wie vorstehend definiert,
- Befüllen der Form mit dem härtbaren Ausgangsmaterial, und danach
- Vernetzenlassen oder Weitervernetzenlassen des härtbaren Ausgangsmaterials,
- Entnahme des vernetzten Materials aus der Form, und danach
- optional Anpassen der Form des vernetzten Materials,
   oder
- Bereitstellen eines erfindungsgemäßen Kits umfassend ein oder mehrere vorgeformte Rohlinge,
- Anpassen der Form eines vorgeformten Rohlings (z.B. durch Abschleifen eines Rohlings mit Übergröße).

Die vorstehend gemachten Ausführungen betreffend bevorzugte Ausgestaltungen der erfindungsgemäßen provisorischen Suprakonstruktion und des erfindungsgemäßen Kits gelten entsprechend auch für das erfindungsgemäße Verfahren.

Ein weiterer Aspekt betrifft die Verwendung eines (Ausgangs-)Materials wie vorstehend definiert zum Herstellen einer provisorischen Suprakonstruktion für ein dentales Implantat oder eines Kerns für einer provisorischen Suprakonstruktion für ein dentales Implantat.

Ein weiterer Aspekt betrifft ein Verfahren zur provisorischen Versorgung eines dentalen Implantats für therapeutische und/oder kosmetische Zwecke, umfassend die Schritte:
(a) Bereitstellen einer erfindungsgemäßen provisorischen Suprakonstruktion wie vorstehend definiert, oder
   Bereitstellen mehrerer provisorischer Suprakonstruktionen wie vorstehend definiert und Auswählen einer provisorischen Suprakonstruktion,
(b) Verbinden der provisorischen Suprakonstruktion mit dem dentalen Implantat und danach
(c) Verstreichenlassen einer gewissen Zeitspanne und danach
(d) Lösen der Verbindung zwischen der provisorischen Suprakonstruktion und dem dentalen Implantat,
und gegebenenfalls danach die folgenden Schritte:
(d) Auswählen einer weiteren in Schritt (a) bereitgestellten provisorischen Suprakonstruktion,
(e) Verbinden der in Schritt (d) ausgewählten provisorischen Suprakonstruktion mit dem dentalen Implantat und danach
(f) Verstreichenlassen einer gewissen Zeitspanne und danach
(g) Lösen der Verbindung zwischen der provisorischen Suprakonstruktion und dem dentalen Implantat,
(h) Wiederholen der Schritte (d) bis (g), bis alle der in Schritt (a) bereitgestellten provisorischen Suprakonstruktionen mit dem Implantat verbunden worden sind und die jeweilige Verbindung wieder gelöst worden ist.

Das erfindungsgemäße Verfahren zur provisorischen Versorgung eines dentalen Implantats für kosmetische Zwecke wird vorzugsweise mit nur einer erfindungsgemäßen provisorischen Suprakonstruktion wie vorstehend definiert durchgeführt.

Bei einem bevorzugten erfindungsgemäßen Verfahren zur provisorischen Versorgung eines dentalen Implantats werden mehrere provisorische Suprakonstruktionen bestehend aus bzw. als Kernmaterial umfassend Materialien mit unterschiedlichen Elastizitätsmoduln bereitgestellt und in der Reihenfolge ansteigender Elastizitätsmoduln zeitlich nacheinander mit dem dentalen Implantat verbunden.

Vorzugsweise wird bei einem erfindungsgemäßen Verfahren zur provisorischen Versorgung eines dentalen Implantats eine solche provisorische Suprakonstruktion mit dem dentalen Implantat verbunden, dass die provisorische Suprakonstruktion bei Kieferschluss in Okklusion mit der Antagonistenbezahnung steht.

Weitere Aspekte der Erfindung ergeben sich aus den nachfolgenden Figuren, Beispielen und den Patentansprüchen.

### Beschreibung der Figuren:

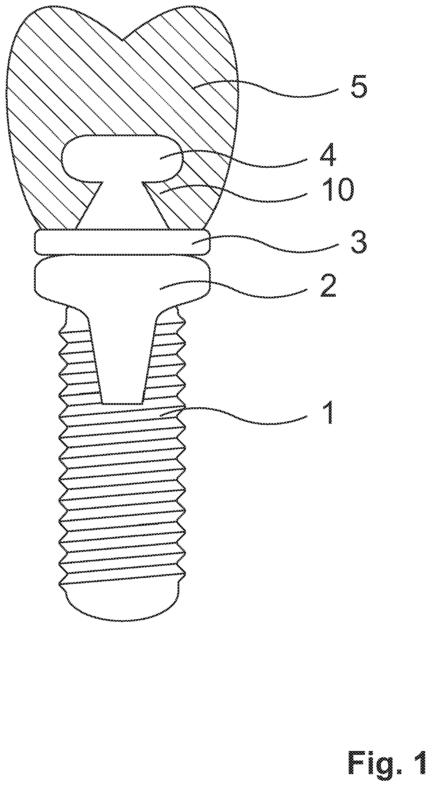

Figur 1 zeigt eine schematische Darstellung einer erfindungsgemäßen provisorischen Suprakonstruktion (5) in der Ausgestaltung einer provisorischen Krone, die mit einem dentalen Implantat (1) unter Einsatz eines Abutments (2, 3, 4) verbunden ist. Ein Element (A) (4) zur Retention der erfindungsgemäßen provisorischen Suprakonstruktion (5) weist Unterschneidungen (10) auf, mittels derer die provisorische Suprakonstruktion formschlüssig befestigt ist in Kraftrichtung entlang der Längsachse des Implantats (1) vom Kieferknochen weg, in den es einzusetzen ist. Element (C) (2) ist als Gingivaformer ausgebildet. Eine Plattform (3) ist als Element (B) des Abutments zur Abstützung der provisorischen Suprakonstruktion (5) in Kraftrichtung entlang der Längsachse des Implantats (1) zum Kieferknochen hin, in den das Implantat einzusetzen ist, vorgesehen.

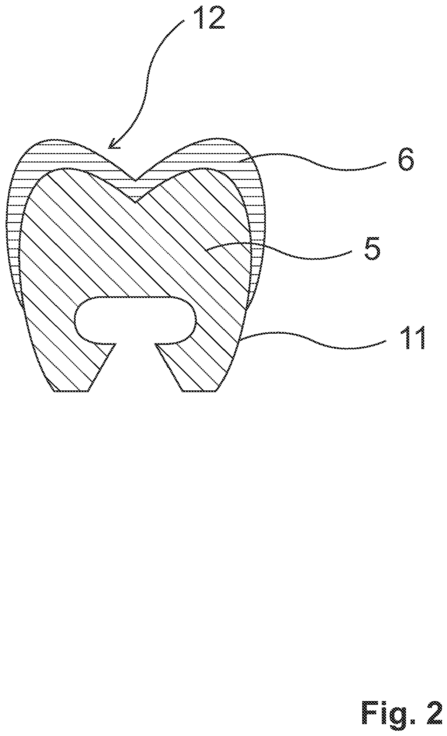

Figur 2 zeigt eine schematische Darstellung einer provisorischen Suprakonstruktion (5, 6) bestehend aus einem Kern (5) und einer Kappe (6) für den Kern. Das Material der Kappe weist einen höheren Elastizitätsmodul auf als das Material des Kerns und besitzt eine hohe Abrasionsfestigkeit. Die Kappe ist so beschaffen, dass sie nur einen Teil der äußeren Oberfläche (11) des Kerns bedeckt, wobei dieser Teil die Kaufläche (12) umfasst.

### Beispiele:

### Beispiel 1: Weiche, mittlere und harte Formulierung

Im Folgenden sind drei verschiedene härtbare Ausgangsmaterialien wiedergegeben, die jeweils aus einer ersten Komponente und einer zweiten Komponente durch Mischen im Volumenverhältnis 1:1 herzustellen sind und die nach der Härtung jeweils ein für eine erfindungsgemäße provisorische Suprakonstruktion geeignetes Material ergeben. Diese drei Materialien sind mit "Formulierung weich", "Formulierung mittel" und "Formulierung hart" gekennzeichnet.

### 1.1 Formulierung weich

**Erste Komponente**

| **Reagenz** | **Anteil [%]** | **Viskosität [mPa*s]** |
|---|---|---|
| Vinylpolysiloxan | 20,00 | 10000 |
| Vinylpolysiloxan | 30,00 | 1000 |
| Vinylpolysiloxan | 8,50 | 200 |
| Methylhydrogenpolysiloxan | 8,10 | 230 |
| Aerosil | 1,00 | |
| Inerter Füllstoff | 32,40 | |
| **Gesamt** | **100,00** | |

**Zweite Komponente**

| **Reagenz** | **Anteil [%]** | **Viskosität [mPa*s]** |
|---|---|---|
| Polymerisationskatalysator | 0,50 | 800 |
| Vinylpolysiloxan | 20,00 | 10000 |
| Vinylpolysiloxan | 35,00 | 1000 |
| Vinylpolysiloxan | 9,00 | 200 |
| Aerosil | 1,50 | |
| Inerter Füllstoff | 34,00 | |
| **Gesamt** | **100,00** | |

### 1.2 Formulierung mittel

**Erste Komponente**

| **Reagenz** | **Anteil [%]** | **Viskosität [mPa*s]** |
|---|---|---|
| Vinylpolysiloxan | 17,00 | 10000 |
| Vinylpolysiloxan | 23,00 | 1000 |
| Vinylpolysiloxan | 7,00 | 200 |
| Methylhydrogenpolysiloxan | 7,00 | 230 |
| Aerosil | 2,00 | |
| Inerter Füllstoff | 44,00 | |
| **Gesamt** | **100,00** | |

**Zweite Komponente**

| **Reagenz** | **Anteil [%]** | **Viskosität [mPa*s]** |
|---|---|---|
| Polymerisationskatalysator | 0,50 | 800 |
| Vinylpolysiloxan | 16,50 | 10000 |
| Vinylpolysiloxan | 28,00 | 1000 |
| Vinylpolysiloxan | 8,20 | 200 |
| Aerosil | 2,50 | |
| Inerter Füllstoff | 44,30 | |
| **Gesamt** | **100,00** | |

### 1.3 Formulierung hart

**Erste Komponente**

| **Reagenz** | **Anteil [%]** | **Viskosität [mPa*s]** |
|---|---|---|
| Vinylpolysiloxan | 8,00 | 10000 |
| Vinylpolysiloxan | 17,00 | 1000 |
| Vinylpolysiloxan | 6,00 | 200 |
| Methylhydrogenpolysiloxan | 5,00 | 230 |
| Aerosil | 2,00 | |
| Inerter Füllstoff | 62,00 | |
| **Gesamt** | **100,00** | |

**Zweite Komponente**

| **Reagenz** | **Anteil [%]** | **Viskosität [mPa*s]** |
|---|---|---|
| Polymerisationskatalysator | 0,50 | 800 |
| Vinylpolysiloxan | 7,00 | 10000 |
| Vinylpolysiloxan | 21,50 | 1000 |
| Vinylpolysiloxan | 6,00 | 200 |
| Aerosil | 3,00 | |
| Inerter Füllstoff | 62,00 | |
| **Gesamt** | **100,00** | |

Die drei Materialien weisen neben einer Dimensionsstabilität (Rückstellvermögen) von > 99% gemäß ISO 4823:2000 die folgenden Eigenschaften auf:

| **Formulierung** | **Shore-Härte** | **Elastizitätsmodul** |
|---|---|---|
| weich | Shore A ca. 25-45 | ca. 27 MPa |
| mittel | Shore A ca. 75-85 | ca. 63 MPa |
| hart | Shore D ca. 35-45 | ca. 110 MPa |

### Beispiel 2: Handanmischung:

Es wurde ein ungefärbtes, zweikomponentiges A-Silicon (Ufi Gel SC gloss, VOCO) mit zahnfarbenen Acrylpulvern (Bosworth Trim®) zur Herstellung von herkömmlichen Kunststoffprovisorien per Hand mit einem Spatel auf einem Wachsblock so lange gemischt, bis Konsistenzen zwischen vollständig flüssig (wenig Pulver) und fast knetbar, aber gerade noch fließfähig (mehr Pulver) entstanden sind. Mit Hilfe dieser Materialien konnten Provisorien der verschiedenen Härtegrade und dementsprechend verschiedenen Elastizitätsmoduln produziert werden.

### Beispiel 3: Kartuschenanmischung:

Es wurde ein dünnfließendes A-Silicon aus der Kartusche (Contrast light, VOCO) mit weiteren Füllstoffen per Hand gemischt, um verschiedene Härtegrade zu bekommen, wobei letztendlich beim fertigen Produkt auch die zusätzlichen Füllstoffe in der Kartusche sein sollen.

### Beispiel 4: Schlauchbeutelanmischung:

Aus Polyether (Impregum®, 3M ESPE) hergestellte Provisorien eignen sich, wenn sie zahnfarben eingefärbt sind. Sie können Kräfte abpuffern (Fig. 8, 9).

## Patentansprüche

1. Provisorische Suprakonstruktion (5,6) für ein dentales Implantat, bestehend aus einem Kern (5) und einer Kappe (6) für den Kern, wobei das Material der Kappe einen höheren Elastizitätsmodul aufweist als das Material des Kerns, wobei der Kern aus einem Material mit einem Elastizitätsmodul im Bereich von 15 bis 200 MPa besteht und
wobei das Material des Kerns eine Shore A-Härte im Bereich von 20 bis 90 oder eine Shore D-Härte im Bereich von 30 bis 50 aufweist und
wobei das Material des Kerns mit einem Elastizitätsmodul im Bereich von 15 bis 200 MPa aus einem gehärteten Ausgangsmaterial besteht, das durch Härtung herstellbar ist, wobei das härtbare Ausgangsmaterial aus folgenden Bestandteilen besteht oder diese umfasst:
(a) ein oder mehrere Silicone umfassend mehratomige vernetzbare einen oder mehrere gegebenenfalls substituierte, vernetzbare Polyether und
(b) einen oder mehrere Katalysatoren oder Starter für die Vernetzung.

2. Provisorische Suprakonstruktion nach Anspruch 1, wobei das härtbare Ausgangsmaterial folgende Bestandteile umfasst:
(c) einen oder mehrere Vernetzer, für die gilt: der oder die Vernetzer sind keine Silicone umfassend mehratomige vernetzbare Gruppen und keine gegebenenfalls substituierten Polyether,
und/oder
(d) einen oder mehrere anorganische oder organische Füllstoffe.

3. Provisorische Suprakonstruktion nach Anspruch 1 oder Anspruch 2, wobei die provisorische Suprakonstruktion eine provisorische Krone ist.

4. Provisorische Suprakonstruktion nach einem der vorangehenden Ansprüche, wobei das härtbare Ausgangsmaterial aus folgenden Bestandteilen besteht oder diese umfasst:
(i)
(a) ein oder mehrere Silicone umfassend Alkenylgruppen die gegebenenfalls substituiert sind,
(b) einen oder mehrere Katalysatoren für die Vernetzung,
(c) ein oder mehrere Organohydrogenpolysiloxane ohne mehratomige vernetzbare Gruppen, und
(d) einen oder mehrere partikuläre anorganische oder organische Füllstoffe,
oder
(ii)
(a) Alkylpolysiloxan mit endständigen Hydroxylgruppen,
(b) einen oder mehrere Katalysatoren,
(c) ein oder mehrere Silane mit zwei oder mehr Alkoxysubstituenten, und
(d) einen oder mehrere partikuläre anorganische oder organische Füllstoffe,
oder
(iii)
(a) ein oder mehrere Polyalkylenglycole, bei denen die endständigen OH-Gruppen durch Aziridin umfassende Gruppen ersetzt sind,
(b) einen oder mehrere Starter, und
(d) einen oder mehrere partikuläre anorganische oder organische Füllstoffe.

5. Provisorische Suprakonstruktion nach einem der vorstehenden Ansprüche, wobei das härtbare Ausgangsmaterial aus folgenden Bestandteilen besteht oder diese umfasst:
(i)
(a) zwei oder mehr Silicone, die jeweils sowohl vernetzbare Gruppen ausgewählt aus der Gruppe bestehend aus Vinyl und Allyl als auch Alkylgruppen umfassen,
(b) einen oder mehrere Katalysatoren umfassend Platin oder Palladium,
(c) Methylhydrogenpolysiloxan, und
(d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe,
oder
(ii)
(a) Polydimethylsiloxan mit endständigen Hydroxylgruppen,
(b) Zinnoctoat und/oder Dibutylzinnlaurat,
(c) ein oder mehrere Silane mit zwei oder mehr Ethoxy-Substituenten, und
(d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe,
oder
(iii)
(a) ein oder mehrere Polyalkylenglycole, bei denen die endständigen OH-Gruppen durch Aziridin umfassende Gruppen ersetzt sind und die zwei unterschiedliche repetitive Einheiten mit zwei bzw. vier C-Atomen aufweisen,
(b) einen oder mehrere kationische Starter, und
(d) oberflächenmodifizierte oder nicht-oberflächenmodifizierte SiO₂-Partikel eines mittleren Teilchendurchmessers von unter 200 nm sowie optional weitere partikuläre anorganische oder organische Füllstoffe.

6. Provisorische Suprakonstruktion nach einem der vorangehenden Ansprüche, wobei der Kern aus einem dauerelastischen Material besteht.

7. Provisorische Suprakonstruktion nach einem der vorangehenden Ansprüche, wobei die Kappe nur einen Teil der äußeren Oberfläche des Kerns bedeckt, wobei dieser Teil die Kaufläche umfasst.

8. Kit zum Herstellen einer oder mehrerer provisorischer Suprakonstruktionen jeweils nach einem der Ansprüche 1 bis 7, umfassend
-
(a) ein oder mehrere Silicone umfassend mehratomige vernetzbare Gruppen und/oder
einen oder mehrere gegebenenfalls substituierte, vernetzbare Polyether,
(b) einen oder mehrere Katalysatoren oder Starter für die Vernetzung
und
(d) einen oder mehrere anorganische oder organische Füllstoffe,
wobei ein oder mehrere farbgebende Bestandteile zur Anpassung der Farbe des einen oder der mehreren Kerne an jeweils eine Farbe natürlicher Zähne vorgesehen sind,
oder
- ein oder mehrere vorgeformte Rohlinge bestehend aus einem gehärteten Ausgangsmaterial umfassend
(a) ein oder mehrere Silicone umfassend mehratomige vernetzbare Gruppen und/oder
einen oder mehrere gegebenenfalls substituierte, vernetzbare Polyether
und
(b) einen oder mehrere Katalysatoren oder Starter für die Vernetzung,
wobei die Farbe des einen bzw. der mehreren vorgeformten Rohlinge jeweils einer Farbe natürlicher Zähne angepasst ist.

9. Kit nach Anspruch 8, umfassend oder bestehend aus einem oder mehreren vorgeformten Rohlingen, deren Form jeweils einer Form natürlicher Zähne angenähert ist.

10. Kit nach Anspruch 8 oder 9, umfassend oder bestehend aus mehreren vorgeformten Rohlingen mit unterschiedlichen Elastizitätsmoduln und/oder Werten der Shore A- bzw. Shore D-Härte.

11. Kit nach einem der Ansprüche 8 bis 10, umfassend oder bestehend aus 3 oder mehr vorgeformten Rohlingen, wobei jeweils ein oder mehrere vorgeformte Rohlinge Elastizitätsmoduln in den Bereichen (i) 15 bis 40 MPa, (ii) 55 bis 70 MPa und (iii) 100 bis 120 MPa. und/oder eine Härte in den Bereichen (i) Shore A-Härte 25 bis 45, (ii) Shore A-Härte 75 bis 85 und (iii) Shore D-Härte 35 bis 45 aufweisen.

12. Kit umfassend oder bestehend aus mehreren (a) provisorischen Suprakonstruktionen nach einem der Ansprüche 1 bis 7, (b) vorgeformten Rohlingen für provisorische Suprakonstruktionen nach einem der Ansprüche 1 bis 7 oder (c) Kombinationen aus jeweils einem Kern für eine provisorische Suprakonstruktion nach einem der Ansprüche 1 bis 7 und einem zugeordneten Kappenrohling.

13. Kit nach Anspruch 12, wobei ein einzelner, mehrere oder sämtliche der (a) provisorischen Suprakonstruktionen, (b) vorgeformten Rohlinge bzw. (c) Kombinationen mit einem Abutment für ein dentales Implantat verbunden sind.

14. Kombination aus einer provisorischen Suprakonstruktion nach einem der Ansprüche 1 bis 7 und einem Abutment zur Befestigung einer provisorischen Suprakonstruktion (5,6) nach einem der Ansprüche 1 bis 7 und/oder einem dentalen Implantat,
wobei das Abutment aus den folgenden Elementen besteht oder diese umfasst:
(A) ein Element (4) zur Retention der provisorischen Suprakonstruktion (5,6),
(B) eine Plattform (3) zur Abstützung der provisorischen Suprakonstruktion (5,6) in Kraftrichtung entlang der Längsachse eines Implantats (1), an dem das Abutment befestigt oder zu befestigen ist zum Kieferknochen hin, in den das Implantat eingesetzt oder einzusetzen ist,
(C) ein Element (2), das als Gingivaformer ausgebildet ist,
wobei Element (4) und die provisorische Suprakonstruktion (5,6) so aneinander angepasst sind, dass die provisorische Suprakonstruktion (5,6) an Element formschlüssig befestigt ist in Kraftrichtung entlang der Längsachse des Implantats (1) vom Kieferknochen weg, in den es eingesetzt oder einzusetzen ist.

15. Verfahren zum Herstellen einer provisorischen Suprakonstruktion nach einem der Ansprüche 1 bis 7 umfassend die Schritte:
- Bereitstellen einer geeigneten Form,
- Bereitstellen eines härtbaren Ausgangsmaterials nach einem der Ansprüche 1, 2, 4 bis 5
- Befüllen der Form mit dem härtbaren Ausgangsmaterial, und danach
- Vernetzenlassen oder Weitervernetzenlassen des härtbaren Ausgangsmaterials,
- Entnahme des vernetzten Materials aus der Form, und danach
- optional Anpassen der Form des vernetzten Materials,
oder
- Bereitstellen eines Kits nach einem der Ansprüche 8 bis 13 umfassend ein oder mehrere vorgeformte Rohlinge,
- Anpassen der Form eines vorgeformten Rohlings.

## Claims

1. Temporary supraconstruction (5,6) for a dental implant, consisting of a core (5) and a cap (6) for the core,
wherein the material of the cap has a higher modulus of elasticity than the material of the core,
wherein the core consists of a material having a modulus of elasticity in the range of from 15 to 200 MPa and
wherein the material of the core has a Shore A hardness in the range of from 20 to 90 or a Shore D hardness in the range of from 30 to 50 and
wherein the material of the core having a modulus of elasticity in the range of from 15 to 200 MPa consists of a cured starting material which can be produced by curing,
wherein the curable starting material consists of the following constituents or comprises these:
(a) one or more silicones containing multi-atom crosslinkable groups and/or one or more optionally substituted, crosslinkable polyethers
and
(b) one or more catalysts or starters for the crosslinking.

2. Temporary supraconstruction according to claim 1, wherein the curable starting material comprises the following constituents:
(c) one or more crosslinking agents wherein: the crosslinking agent or agents are not silicones containing multi-atom crosslinkable groups and not optionally substituted polyethers,
and/or
(d) one or more inorganic or organic fillers.

3. Temporary supraconstruction according to claim 1 or claim 2, wherein the temporary supraconstruction is a temporary crown.

4. Temporary supraconstruction according to one of the preceding claims, wherein the curable starting material consists of the following constituents or comprises these:
(i)
(a) one or more silicones containing alkenyl groups which are optionally substituted,
(b) one or more catalysts for the crosslinking,
(c) one or more organohydrogenpolysiloxanes without multi-atom crosslinkable groups, and
(d) one or more particulate inorganic or organic fillers,
or
(ii)
(a) alkylpolysiloxane with terminal hydroxyl groups,
(b) one or more catalysts,
(c) one or more silanes with two or more alkoxy substituents, and
(d) one or more particulate inorganic or organic fillers,
or
(iii)
(a) one or more polyalkylene glycols in which the terminal OH groups are replaced by groups containing aziridine,
(b) one or more starters, and
(d) one or more particulate inorganic or organic fillers.

5. Temporary supraconstruction according to one of the preceding claims, wherein the curable starting material consists of the following constituents or comprises these:
(i)
(a) two or more silicones which each contain both crosslinkable group selected from the group consisting of vinyl and allyl, and alkyl groups,
(b) one or more catalysts containing platinum or palladium,
(c) methylhydrogenpolysiloxane, and
(d) surface modified or non-surface modified SiO₂ particles having an average particle diameter of less than 200 nm and optionally further particulate inorganic or organic fillers,
or
(ii)
(a) polydimethylsiloxane with terminal hydroxyl groups,
(b) tin octoate and/or dibutyltin laurate,
(c) one or more silanes with two or more ethoxy substituents, and
(d) surface modified or non-surface modified SiO₂ particles having an average particle diameter of less than 200 nm and optionally further particulate inorganic or organic fillers,
or
(iii)
(a) one or more polyalkylene glycols in which the terminal OH groups are replaced by groups containing aziridine and which have two different repetitive units with two or, respectively, four C atoms,
(b) one or more cationic starters, and
(c) surface modified or non-surface modified SiO₂ particles having an average particle diameter of less than 200 nm and optionally further particulate inorganic or organic fillers.

6. Temporary supraconstruction according to one of the preceding claims, wherein the core consists of a permanently flexible material.

7. Temporary supraconstruction according to one of the preceding claims, wherein the cap covers only a part of the outer surface of the core, wherein this part contains the chewing area.

8. Kit for producing one or more temporary supraconstructions each according to one of claims 1 to 7, comprising
-
(a) one or more silicones containing multi-atom crosslinkable groups and/or one or more optionally substituted, crosslinkable polyethers
(b) one or more catalysts or starters for the crosslinking and
(d) one or more inorganic or organic fillers,
wherein one or more colour-imparting constituents are provided for matching the colour of the one or more cores in each case to a colour of natural teeth,
or
- one or more preformed blanks consisting of a cured starting material comprising
(a) one or more silicones containing multi-atom crosslinkable groups and/or
one or more optionally substituted, crosslinkable polyethers
and
(b) one or more catalysts or starters for the crosslinking,
wherein the colour of the one or more preformed blanks is matched in each case to a colour of natural teeth.

9. Kit according to claim 8, comprising or consisting of one or more preformed blanks, the shape of which in each case is approximately a shape of natural teeth.

10. Kit according to claim 8 or 9, comprising or consisting of several preformed blanks having different moduli of elasticity and/or Shore A or Shore D hardness values.

11. Kit according to one of claims 8 to 10, comprising or consisting of 3 or more preformed blanks, wherein in each case one or more preformed blanks have moduli of elasticity in the ranges (i) 15 to 40 MPa, (ii) 55 to 70 MPa and (iii) 100 to 120 MPa, and/or a hardness in the ranges (i) Shore A hardness 25 to 45, (ii) Shore A hardness 75 to 85 and (iii) Shore D hardness 35 to 45.

12. Kit comprising or consisting of several (a) temporary supraconstructions according to one of claims 1 to 7, (b) preformed blanks for temporary supraconstructions according to one of claims 1 to 7 or (c) combinations of in each case a core for a temporary supraconstruction according to one of claims 1 to 7 and an associated cap blank.

13. Kit according to claim 12, wherein an individual, several or all of the (a) temporary supraconstructions, (b) preformed blanks or, respectively, (c) combinations are combined with an abutment for a dental implant.

14. Combination of a temporary supraconstruction according to one of claims 1 to 7 and an abutment for fixing a temporary supraconstruction (5,6) according to one of claims 1 to 7 and/or a dental implant,
wherein the abutment consists of the following elements or comprises these:
(A) an element (4) for retention of the temporary supraconstruction (5,6)
(B) a platform (3) for supporting the temporary supraconstruction (5,6) in the force direction along the longitudinal axis of an implant (1), to which the abutment is fixed or to be fixed, towards the jaw bone into which the implant is inserted or to be inserted,
(C) an element (2) which is constructed as a gingiva former,
wherein the element (4) and the temporary supraconstruction (5,6) are matched to one another such that the temporary supraconstruction (5,6) is fixed to the element with positive locking in the force direction along the longitudinal axis of the implant (1) away from the jaw bone into which it is inserted or to be inserted.

15. Method for producing a temporary supraconstruction according to one of claims 1 to 7, comprising the steps:
- providing a suitable mould,
- providing a curable starting material according to one of claims 1, 2, 4 to 5,
- filling the mould with the curable starting material, and thereafter
- allowing the curable starting material to crosslink or crosslink further,
- removing the crosslinked material from the mould, and thereafter
- optionally adapting the shape of the crosslinked material
or
- providing a kit according to one of claims 8 to 13 comprising one or more preformed blanks,
- adapting the shape of a preformed blank.

## Revendications

1. Superstructure provisoire (5, 6) pour un implant dentaire, consistant en un noyau (5) et une coiffe (6) pour le noyau, dans laquelle le matériau de la coiffe présente un module d'élasticité supérieur au matériau du noyau, dans laquelle le noyau consiste en un matériau présentant un module d'élasticité dans la plage de 15 à 200 MPa et
dans laquelle le matériau du noyau présente une dureté Shore A dans la plage de 20 à 90 ou une dureté Shore D dans la plage de 30 à 50 et
dans laquelle le matériau du noyau ayant un module d'élasticité dans la plage de 15 à 200 MPa consiste en un matériau de départ durci qui peut être produit par durcissement,
dans laquelle le matériau de départ durcissable consiste en matériaux suivants ou comprend ceux-ci :
(a) une ou plusieurs silicone(s) comprenant des groupes polyatomiques réticulables et/ou
un ou plusieurs polyéthers réticulables, éventuellement substitués
et
(b) un ou plusieurs catalyseurs ou initiateur(s) de réticulation.

2. Superstructure provisoire selon la revendication 1, dans laquelle le matériau de départ durcissable comprend les composants suivants :
(c) un ou plusieurs agents de réticulation satisfaisant à : le ou les agents de réticulation ne sont pas des silicones comprenant des groupes polyatomiques réticulables et ne sont pas des polyéthers éventuellement substitués, et/ou
(d) un ou plusieurs agents de charge inorganiques ou organiques.

3. Superstructure provisoire selon la revendication 1 ou la revendication 2, dans laquelle la superstructure provisoire est une couronne provisoire.

4. Superstructure provisoire selon l'une des revendications précédentes, dans laquelle le matériau de départ durcissable consiste en composants suivants ou comprend ceux-ci
(i)
(a) une ou plusieurs silicone(s) comprenant des groupes alcényle qui sont éventuellement substitués,
(b) un ou plusieurs catalyseurs pour la réticulation,
(c) un ou plusieurs organohydrogénopolysiloxanes sans groupes polyatomiques réticulables, et
(d) un ou plusieurs agents de charge particulaires, inorganiques ou organiques,
ou
(ii)
(a) un alkylpolysiloxane comportant des groupes hydroxyle terminaux
(b) un ou plusieurs catalyseurs,
(c) un ou plusieurs silanes comportant deux ou plusieurs substituants alcoxy, et
(d) un ou plusieurs agents de charge particulaires inorganiques ou organiques,
ou
(iii)
(a) un ou plusieurs polyalkylène glycols, dans lesquels les groupes OH terminaux sont remplacés par des groupes comprenant une aziridine,
(b) un ou plusieurs initiateurs, et
(d) un ou plusieurs agents de charge particulaires inorganiques ou organiques.

5. Superstructure provisoire selon l'une des revendications précédentes, dans laquelle le matériau de départ durcissable consiste en composants suivants ou comprend ceux-ci :
(i)
(a) deux ou plusieurs silicones qui comprennent respectivement aussi bien des groupes réticulables choisis dans le groupe consistant en vinyle et allyle qu'également des groupes alkyle,
(b) un ou plusieurs catalyseurs comprenant le platine ou le palladium,
(c) du méthylhydrogénopolysiloxane, et
(d) des particules de SiO₂ modifiées en surface ou non modifiées en surface, d'un diamètre particulaire moyen de moins de 200 nm et éventuellement d'autres agents de charge particulaires inorganiques ou organiques,
ou
(ii)
(a) du polydiméthylsiloxane comportant des groupes hydroxyle terminaux,
(b) de l'octate d'étain et/ou du laurate de dibutylétain,
(c) un ou plusieurs silanes comportant deux ou plusieurs substituants éthoxy,
et
(d) des particules de SiO₂ modifiées en surface ou non modifiées en surface, d'un diamètre particulaire moyen de moins de 200 nm et éventuellement d'autres agents de charge particulaires inorganiques ou organiques,
ou
(iii)
(a) un ou plusieurs polyalkylène glycols, dans lesquels les groupes OH terminaux sont remplacés par des groupes comprenant une aziridine et les deux motifs répétitifs différents présentent deux à quatre atomes de carbone,
(b) un ou plusieurs initiateurs cationiques, et
(d) des particules de SiO₂ modifiées en surface ou non modifiées en surface, d'un diamètre particulaire moyen de moins de 200 nm et éventuellement d'autres agents de charge particulaires inorganiques ou organiques.

6. Superstructure provisoire selon l'une des revendications précédentes, dans laquelle le noyau consiste en un matériau durablement élastique.

7. Superstructure provisoire selon l'une des revendications précédentes, dans laquelle la coiffe recouvre uniquement une partie de la surface extérieure du noyau, dans laquelle cette partie comprend la surface de mastication.

8. Kit pour la fabrication d'une ou plusieurs superstructures provisoires, respectivement selon l'une des revendications 1 à 7, comprenant
-
(a) une ou plusieurs silicones comprenant des groupes polyatomiques réticulables et/ou
un ou plusieurs polyéthers réticulables, éventuellement substitués
(b) un ou plusieurs catalyseurs ou initiateurs de réticulation et
(d) un ou plusieurs agents de charge inorganiques ou organiques,
dans lequel un ou plusieurs composants teintants sont prévus pour l'ajustement de la teinte d'un ou plusieurs noyaux à respectivement une teinte des dents naturelles,
ou
- une ou plusieurs ébauches préformées consistant en un matériau de départ durci comprenant
(a) une ou plusieurs silicone(s) comprenant des groupes polyatomiques réticulables et/ou
un ou plusieurs polyéthers réticulables, éventuellement substitués
et
(b) un ou plusieurs catalyseurs ou initiateurs de réticulation,
dans lequel la teinte de l'une ou des plusieurs ébauches préformées est ajustée à respectivement une teinte des dents naturelles.

9. Kit selon la revendication 8, comprenant ou consistant en une ou plusieurs ébauches préformées, dont la forme s'approche respectivement d'une forme de dents naturelles.

10. Kit selon la revendication 8 ou 9, comprenant ou consistant en plusieurs ébauches préformées comportant des modules d'élasticité différents et/ou des valeurs Shore A ou Shore D différentes.

11. Kit selon l'une des revendications 8 à 10, comprenant ou consistant en 3 ébauches préformées ou plus, dans lequel respectivement une ou plusieurs ébauches préformées présentent des modules d'élasticité dans les plages de (i) 15 à 40 MPa, (ii) 55 à 70 MPa et (iii) 100 à 120 MPa, et/ou une dureté dans les plages de (i) dureté Shore A de 25 à 45, (ii) dureté Shore A de 75 à 85 et (iii) dureté Shore D de 35 à 45.

12. Kit comprenant ou consistant en plusieurs (a) superstructures provisoires selon l'une des revendications 1 à 7, (b) des ébauches préformées pour des superstructures provisoires selon l'une des revendications 1 à 7 ou (c) des combinaisons de respectivement un noyau pour une superstructure provisoire selon l'une des revendications 1 à 7 et une ébauche de coiffe associée.

13. Kit selon la revendication 12, dans lequel une, plusieurs ou toutes parmi (a) les superstructures provisoires, (b) les ébauches préformées ou (c) les combinaisons sont reliées à un pilier pour un implant dentaire.

14. Combinaison d'une superstructure provisoire selon l'une des revendications 1 à 7 et d'un pilier pour la fixation d'une superstructure provisoire (5, 6) selon l'une des revendications 1 à 7 et/ou d'un implant dentaire,
dans lequel le pilier consiste en les éléments suivants ou comprend ceux-ci :
(A) un élément (4) pour retenir la superstructure provisoire (5, 6),
(B) une plateforme (3) pour le soutien de la superstructure provisoire (5, 6) dans la direction de la force le long de l'axe longitudinal d'un implant (1) sur lequel le pilier est fixé ou est à fixer, vers l'os maxillaire, dans lequel l'implant est inséré ou est à insérer,
(C) un élément (2) conçu comme pilier de cicatrisation,
dans lequel l'élément (4) et la superstructure provisoire (5, 6) sont ajustés l'un à l'autre de telle sorte que la superstructure provisoire (5,6) soit fixée à l'élément par ajustement de formes dans la direction de la force le long de l'axe longitudinal de l'implant (1) s'éloignant de l'os maxillaire dans lequel il est inséré ou est à insérer.

15. Procédé de fabrication d'une superstructure provisoire selon l'une des revendications 1 à 7, comprenant les étapes de :
- mise à disposition d'une forme appropriée,
- mise à disposition d'un matériau de départ durcissable selon l'une des revendications 1, 2, 4 à 5
- remplissage de la forme avec le matériau de départ durcissable, et ensuite
- réticulation ou poursuite de la réticulation du matériau de départ durcissable,
- retrait du matériau réticulé de la forme, et ensuite
- ajustement éventuel de la forme du matériau réticulé, ou
- mise à disposition d'un kit selon l'une des revendications 8 à 13 comprenant une ou plusieurs ébauches préformée,
- ajustement de la forme d'une ébauche préformée.
